Europäisches Patentamt

European Patent Office

Office européen des brevets

(11) Publication number: **0 127 908**
**B1**

# EUROPEAN PATENT SPECIFICATION

(45) Date of publication of patent specification: **31.05.89**

(21) Application number: **84200545.6**

(22) Date of filing: **17.04.84**

(51) Int. Cl.⁴: **G 01 N 33/28**

(54) Method and apparatus for analyzing a hydrocarbon sample.

(30) Priority: **31.05.83 US 499122**

(43) Date of publication of application:
**12.12.84 Bulletin 84/50**

(45) Publication of the grant of the patent:
**31.05.89 Bulletin 89/22**

(84) Designated Contracting States:
**DE FR GB IT NL**

(56) References cited:
**GB-A-1 252 442**
**US-A- 386 562**
**US-A-3 550 428**
**US-A-4 287 752**
**US-A-4 384 471**

(73) Proprietor: **SHELL INTERNATIONALE**
**RESEARCH MAATSCHAPPIJ B.V.**
**Carel van Bylandtlaan 30**
**NL-2596 HR Den Haag (NL)**

(72) Inventor: **Szakasits, Julius Jozsef**
**11614 Knobcrest**
**Houston Texas 77070 (US)**
Inventor: **Robinson, Robert Eugene**
**11631 Jaycreek**
**Houston Texas 77070 (US)**

(74) Representative: **Aalbers, Onno et al**
**P.O. Box 302**
**NL-2501 CH The Hague (NL)**

Courier Press, Leamington Spa, England.

# Description

This invention relates generally to gas chromatography and, more particularly, to the paraffin, naphthene and aromatic analysis of reformer feedstock and product.

Chromatographic analysis of mixtures of paraffins, naphthenes and aromatics (PNA-analysis) is disclosed in the article "Better gasoline chromatography" by H. Boer and P. van Arkel in "Hydrocarbon Processing" February 1972, pages 80—84. Such analysis is of importance for control and optimization of reforming and platforming processes running on feed of varying composition.

Frequent changes in feedstock quality for catalytic reformers have created a need for an on site instrument which can quickly provide information on feed and product composition changes to assist in plant surveillance and process tuning. The failure to recognize poor plant performance or upset at an early stage can lead to a serious loss in reformer yields and/or a premature need for catalyst regeneration. In the past characterizing of reformer feeds and products has generally been done by a combination of gas chromatography and analytical chemistry methods in a central laboratory which is usually remote from the refinery. This technique is time consuming and often leads to a sizable delay between the times the sample is taken and the results are made available to the refinery.

Therefore, it is an object of the present invention to provide a method of an apparatus for analyzing a hydrocarbon sample from a reformer process to provide a rapid and detailed analysis of the paraffin, naphthene and aromatic composition of the sample.

The apparatus of the invention for analyzing a hydrocarbon sample comprising paraffins, naphthenes and aromatics, comprises means for providing a sample; a first means for supplying gas connected to said sample providing means, a first valve means connected to said sample providing means, a polar column connected to said first valve means for retarding the aromatics of said sample while the remainder passes through; a 13X molecular sieve coated open tubular column (13X MSCOT column) comprising an elongated capillary tube and a layer comprising a plurality of disunited particles of 13X material disposed on the inner surface of the tube by filling the tube with a suspension comprising a dispersive liquid and 13X materials and flowing an inert gas through the tube to dry the tube thereby leaving said layer attached to the inner surface of said tube for separating the paraffins and naphthenes in said sample by carbon number, said 13X MSCOT column having a first end connected to said first valve means and a second end connected to means for determining the amounts of paraffins and naphthenes separated by carbon number by said 13X MSCOT column; means for varying the temperature of said 13X MSCOT column; a mixed binary phase column for separating said sample by components from $C_1$ to at least $C_8$, said mixed binary phase column having a first end connected to said first valve means and a second end connected to means for determining the amounts of said sample separated by components from $C_1$ through at least $C_8$ by said mixed binary phase column; means for maintaining said mixed binary phase column at a predetermined temperature; a nonplanar stationary phase column for separating the aromatics in said sample, said nonpolar stationary phase column having a first end connected to said first valve means and a second end connected to means for determining the amounts of said aromatics separated by said nonpolar stationary phase column; means for varying the temperature of said nonpolar stationary phase column; a second means for supplying gas connected to said first valve means; a third means for supplying gas connected to said first valve means; said first valve means having at least a first and second position, such that in said first position, said polar column is connected to an injecting means, said 13X MSCOT column is connected to said polar column such that a portion of the effluent from said polar column passes through said 13X MSCOT column and said mixed binary phase column is connected to said polar column such that at least a portion of the effluent from said polar column passes through said mixed binary phase column and such that in said second position the flow through said polar column is reversed, said polar column is connected to said first end of said nonpolar stationary column such that the flow from said polar column enters said first end of said nonpolar stationary column, said first end of said 13X MSCOT column is connected to said second gas supply means, and said first end of said mixed binary phase column is connected to said third gas supply means.

The method of the present invention of analyzing a hydrocarbon sample comprising paraffins, naphthenes and aromatics, comprises the steps of: injecting a hydrocarbon sample into a first stream of carrier gas; passing the first stream of carrier gas and sample through a polar column having a material for retarding aromatics from the remainder of the sample; retarding in the polar column the aromatics from the sample and allowing the remainder of the sample and the first stream of carrier gas to pass through thereby providing a first effluent from the polar column; splitting the first effluent from the polar column into at least first and second portions; passing the first effluent portion through a 13X molecular sieve coated open tubular column (13X MSCOT column) having a layer comprising a plurality of dis-united particles of 13X material disposed on an inner surface of the column by filling the column with a suspension comprising a dispersive liquid and 13X material and flowing an inert gas through the column thereby leaving a layer comprising a plurality of disunited particles of the 13X material attached to the inner surface of the column for separating paraffins and naphthenes by carbon number; passing the second effluent portion through a mixed binary phase column having a material for separating the second effluent portion by components from $C_1$ through at least $C_8$; separating in said mixed binary phase column the second effluent portion by components from $C_1$ through at least $C_8$; reversing the flow of said first stream of carrier gas through said polar column to remove the aromatics from the polar column thereby providing a second effluent from the polar column; passing the second effluent through a nonpolar stationary phase column having a material for separating the aromatics; separating in said nonpolar stationary phase column the aromatics from the second effluent; providing a second stream of carrier gas to the 13X MSCOT column; providing a third stream of carrier gas to the mixed binary phase column; modifying the temperature of the 13X MSCOT column; modifying the temperature of the nonpolar stationary phase column; detecting the amounts of paraffins and naphthenes separated by carbon number from said sample in said 13X MSCOT column; detecting the amounts of components from $C_1$ through at least $C_8$ separated from said sample in said mixed binary phase column; and detecting the amounts of aromatics separated from said sample in said nonpolar stationary phase column.

It is remarked that US—A—3,550,428 discloses a hydrocarbon separation and analysis method and apparatus utilizing gas-liquid chromatography.

Further, US—A—4,384,471 discloses a chromatographic analysis of hydrocarbon mixtures wherein paraffin, olefin and aromatic compounds in a gasoline containing hydrocarbons up to about $C_{13}$ can be analyzed and identified by gas-liquid chromatography.

US—A—4,287,752 discloses a method and apparatus for analyzing the composition of a gaoline-range petroleum product using a copper on silica gel adsorption composition, and GB—A—1,252,442 discloses a hydrocarbon separation process by means of molecular sieves. However, the present invention cannot be derived from these citations.

The apparatus and method of the present invention provide a detailed analysis in approximately two hours of the paraffin, naphthene and aromatic breakdown of a hydrocarbon sample boiling below 225°C, including the $C_5/C_6$ ring naphthenes distribution. The data provided by the present invention can be used, for example, for inputting to a plant model to determine the steps that are necessary to optimize the reforming process. A plurality of columns and detectors are used concurrently to expand the analyzer's scope and to shorten the analysis time. In an alternative embodiment, the present invention can be provided with means for splitting the sample before it is passed through the polar column. A portion of the sample is provided to a column having a material for separating a predetermined inorganic.

component of the sample and means for detecting the amount of such component separated by the column.

The present invention will now be described in more detail by way of example with reference to the accompanying drawings, in which:

Fig. 1 represents a diagrammatic view of a reformer process analyzer according to the present invention;

Fig. 1A represents a detail of the analyzer of Fig. 1;

Fig. 2 represents a chromatogram of the component by component separation of a typical hydrocarbon sample on the mixed binary phase column;

Fig. 3 represents a chromatogram of the paraffin-naphthene separation of a typical hydrocarbon sample on the 13X MSCOT column;

Fig. 4 represents a chromatogram showing the separation of the aromatics from a typical hydrocarbon sample on the nonpolar stationary phase column;

Fig. 5 represents an alternative embodiment of the present invention.

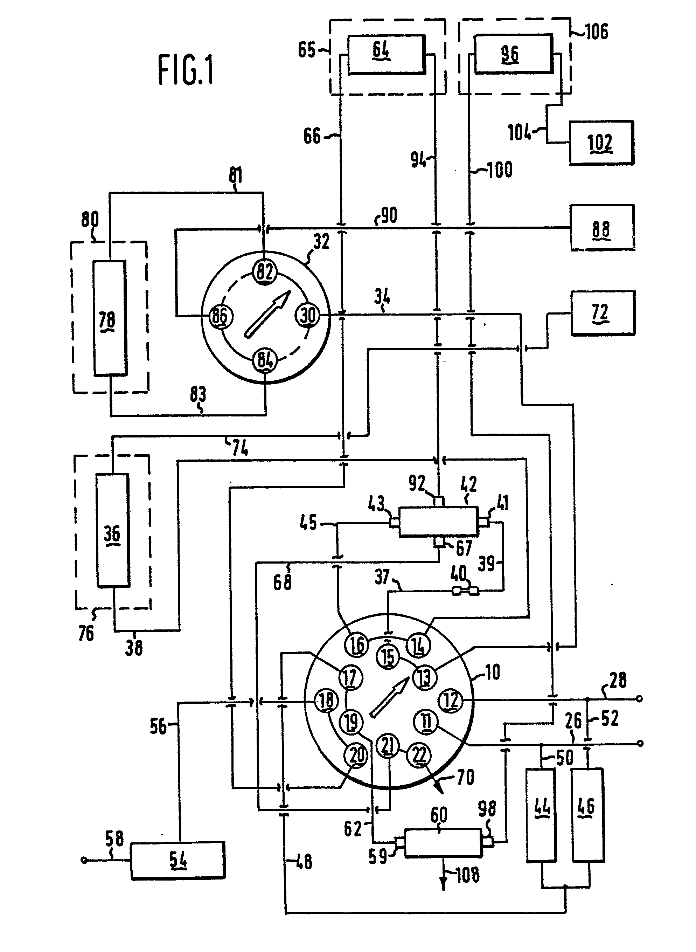

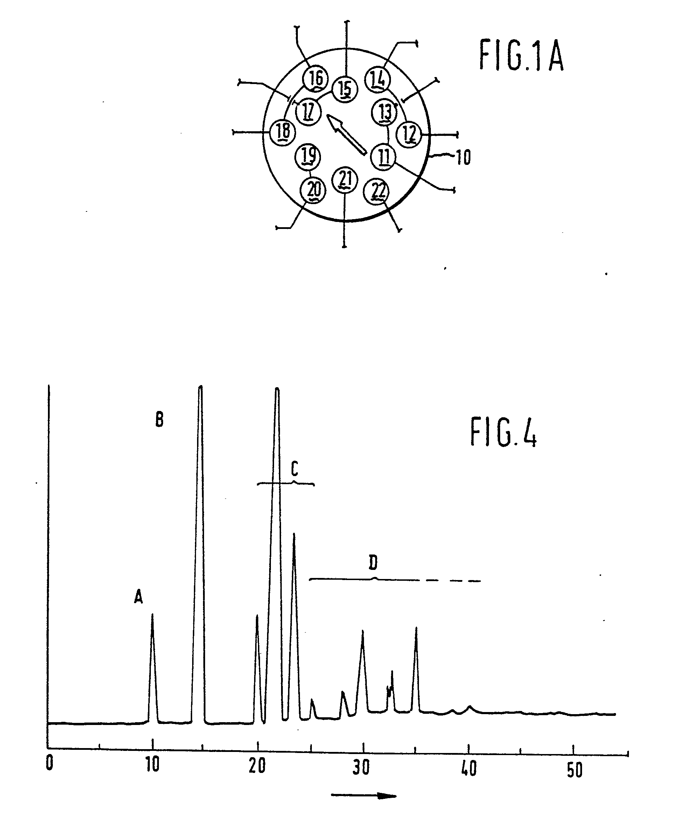

Referring now to Fig. 1 an embodiment of the chromatographic analyzer of the present invention is illustrated. The analyzer has a valve 10 which has twelve ports labelled 11 through 22. The valve 10 has two positions in which the ports are connected internally in different configurations. When the valve 10 is in the first position the port 13 is connected to the port 15, the port 14 is connected to the port 16, the port 17 is connected to the port 19, the port 18 is connected to the port 20, the port 21 is connected to the port 22, and the ports 11 and 12 are not connected to any of the other ports. When the valve 10 is switched to the second position as illustrated in Fig. 1A, the port 11 is connected to the port 13, the port 12 is connected to the port 14, the port 15 is connected to the port 17, the port 16 is connected to the port 18, the port 19 is connected to the port 20 and the ports 21 and 22 are not connected to any of the other ports. The port 11 is connected to a line 26 which is adapted to be connected to an inert gas supply such as helium. The port 12 is connected to a line 28 which is adapted to be connected to an inert gas supply, such as helium.

The port 13 is connected to a port 30 of a valve 32 by a line 34. The port 14 is connected to one side of a 13X MSCOT column 36 by a line 38. The port 15 is connected by a line 37 to a flow restrictor 40. The other end of the flow restrictor 40 is connected to a port 41 of a splitter 42 by a line 39. The port 43 of a splitter 42 is connected to the port 16 of the valve 10 by a line 45. The port 17 is connected to the parallel combination of the valves 44 and 46 by a line 48. The other end of the valve 44 is connected to the line 26 by a line 50, and the other end of the valve 46 is connected to the line 28 by a line 52. The port 18 is connected to a sample injector 54 by a line 56. A line 58 which is adapted to be connected to an inert gas supply, such as helium or argon, is connected to the injector 54 which can be either a manual or automatic injector. In an advantageous embodiment the injector 54 has a glass liner to minimize decomposition and adsorption of sensitive materials of the sample on the hot metal surface of the injector. The port 19 is connected to a port 59 of a splitter 60 by a line 62. The port 20 is connected to one end of a polar column 64 by a line 66. The port 21 is connected to a port 67 of the splitter 42 by a line 68. The port 22 is vented to atmosphere by a line 70.

The column 36 is a 13X molecular sieve coated open tubular column. Finely ground 13X molecular sieve material is combined with water to form a slurry which is pressured through a clean length of stainless steel capillary tubing. A purge is maintained to dry the column, thereby leaving the 13X sieve particles attached to the wall of the tubing. The finely divided particle layer provides a high resolution, type separation of naphthenes, iso-paraffins and paraffins boiling up to 255°C. A suspension of the 13X particles in the water can be achieved by means of stirring or vibrating the slurry. Usually, a treatment of two or three minutes is sufficient if no additional comminution of the solid is desired.

Before the slurry or suspension is pressed into the capillary tube, it is advantageous to pass it through a pressure sieve in order to eliminate coarser agglomerates which could lead to clogging of the capillary tube. An advantageous manner of separating the desired sized particles is to allow the larger particles to settle during a predetermined time interval in the range of 30—120 minutes. One half to two-thirds of the upper portion of the slurry is then pipetted into a separate container. The capillary is filled by means of a pressure vessel and an inert gas, such as nitrogen, helium, argon and so forth. The pressure to be used depends on the diameter and the length of the capillary and on the viscosity of the suspension; usually the pressure is between 27.5—82.5 kPa (4—12 psig). The capillary tube should be cleansed from impurities by washing it thoroughly, such as by using 0.1—0.3 N HCl followed by water which is followed by acetone and finally water again, prior to filling. The purging of the tube is performed by flowing an inert gas, such as helium, through the tube to dry it thereby leaving a layer of disunited 13X particles attached to the inner surface or wall of the tube. Usually a purge of 0.1—1 ml/min is maintained for approximately two to five hours to dry the tube. Next, the column is stabilized at 420—440°C for about twice as long as the drying period. In an advantageous embodiment the tube consists of 15 metres of a stainless steel capillary tube with an inner diameter of 0.5 millimetres and contains approximately 1 mg of 13X molceular sieve material having a particle size in the range of 0.4—12.0 microns. One end of the column 36 is connected to the port 14 of the valve 10 by the line 38, and the other end is connected to a flame ionization detector 72 by a line 74. The column 36 is positioned in a temperature programmable oven 76 which is indicated by dotted lines. The temperature of the oven 76 can be programmed by, for example, a suitable computer/controller, to provide suitable heating of the column 36.

The column 78 is a mixed binary phase column for separating the sample by components from $C_1$ through at least $C_8$. Generally, the $C_9{}^+$ materials are backflushed by the valve 32. In an advantageous embodiment, the column 78 is an n-hexadecane/Fluorolube Oil LG-160 (FLO) capillary column having a film thickness of approximately 0.7 micrometres and is made of 30 metres of stainless steel capillary tubing having an inside diameter of 0.25 millimetres. An advantageous concentration is 4.35 parts of n-hexadecane and 1 part FLO by volume. The column 78 is positioned in an isothermal oven 80 which is maintained at a temperature of, for example, 18.5°C. One end of the column 78 is connected to a port 82 of the valve 32 by a line 81, and the other end of the column 78 is connected to a port 84 of the valve 32 by a line 83. The port 86 of the valve 32 is connected to a flame ionization detector 88 by a line 90. The valve 32 has two positions in which the ports are connected internally in different configurations. In the first position the ports 30 and 82 are connected and the ports 84 and 86 are connected. In the second position, as indicated by the dotted lines, the ports 30 and 84 are connected, and the ports 82 and 86 are connected. Switching the valve 32 from one position to the other reverses the flow through the column 78.

The column 64 is a polar column for separating the aromatics from the remainder of the sample. In an advantageous embodiment the column 64 is a stainless steel tube having a length of 60 centimetres and an inside diameter of 2.3 millimetres, with BC-120 stationary phase, on Chromosorb-P AW Dimethylchlorosilane to make the saturates from aromatic separation. The mesh particle size is 100/120 M. One end of the column 64 is connected to the port 20 of the valve 10 by a line 66, and the other end of the column 64 is connected to the port 92 of the splitter 42 by a line 94. The column 64 is positioned in the oven 65, which is indicated by dotted lines, and is maintained at a constant temperature. The column 96 is a nonpolar stationary phase column for separating the aromatics in the sample. In an advantageous embodiment the column 96 is a stainless steel support coated open tubular column having a length of 30 metres and an inside diameter of 0.5 millimetres which uses squalane for the separation. One end of the column 96 is connected to the port 98 of the splitter 60 by a line 100, and the other end of the column 96 is connected to the flame ionization detector 102 by a line 104. The column 96 is located in a temperature programmable oven 106 which is indicated by dotted lines. The temperature of the oven 106 is varied during the analysis. If desired, the columns 64 and 96 can be located in the same oven. The splitter 60 has an additional port 108 which is vented to atmosphere; the port 108 can include a flow restrictor to limit the flow to atmosphere. The flame ionization detectors 72, 88 and 102 can include chart recorders and/or electronic circuitry to provide a signal suitable for inputting to a computer/controller. The operation of the analyzer, including the operation of the valves, the temperature programming of the ovens, and the output recording from the detectors, can be under the control of a master computer/controller. This master computer/controller can also include functions for data manipulation and output formatting, such as the calculation of the paraffins, naphthenes and aromatics and the $C_5/C_6$ ring napthenes distribution as is known in the art.

The operation of the reformer process analyzer of the present invention can be described as follows. The sample is injected into the analyzer by the injector 54. The carrier gas flow from the line 58 carries the sample from the injector 54 through the line 56 to the port 18 of the valve 10. The sample then passes from the port 18 to the port 20 from which it passes through the line 66 to the column 64. The column 64 retards the aromatics of the sample and allows the remainder of the sample and carrier gas to pass through the line 94. The line 94 provides the remainder of the sample to the port 92 of the splitter 42. The splitter 42 splits the remainder of the sample three ways such that one portion passes through the port 43 of the splitter 42, through the line 45 to the port 16 of the valve 10 to the port 14 from which it passes through the line 38 to the column 36. A second portion of the sample passes through the port 67 of the splitter 42 to the line 68 which is connected to the port 21 of the valve 10. The sample passes from the port 21 to the port 22 from which it is exhausted to atmosphere by the line 70. The third portion of the sample passes through the port 41 of the splitter 42, the line 39, the restrictor 40 to the line 37. The sample passes through the line 37 to the port 15 of the valve 10 and is exhausted therefrom by the port 13 to the line 34. The sample passes through the line 34 to the port 30 of the valve 32 and is exhausted therefrom at the port 82 into the line 81 and hence the column 78. The sample passes through the column 78, and the effluent from the column 78 passes through the line 83 to the valve 30 at the port 84 and is exhausted therefrom at the port 86 into the line 90 to the flame ionization detector 88. The carrier gas from the lines 26 and 28 flows into the line 50 and the valve 44 and the line 52 and the valve 46 respectively. The combined flow then passes through the line 48 to the port 17 and is exhausted from the valve 10 at the port 19. The line 62 provides the carrier gas to the port 59 of the splitter 60. A portion of the carrier gas is exhausted to atmosphere by the line 108 and the remainder passes through the port 98 of the splitter 60 to the line 100. From the line 100 the carrier gas passes through the column 96, thus purging it, and passes through the line 104 to the flame ionization detector 102. Before the valve 10 is switched from its first position the valves 44 and 46 are closed in a predetermined time sequence to raise the carrier gas pressure slightly, 13.7—34.5 kPa (2—5 psi), above normal operating pressures in the lines 26 and 28 after the valve 10 is switched to its second position. This increased pressure prevents diffusing of the sample from the columns 36 and 78 into the lines 38 and 81 respectively. For optimum separation two different molecular weight carrier gases should be used, such as argon in the line 58 and helium in the lines 26 and 28.

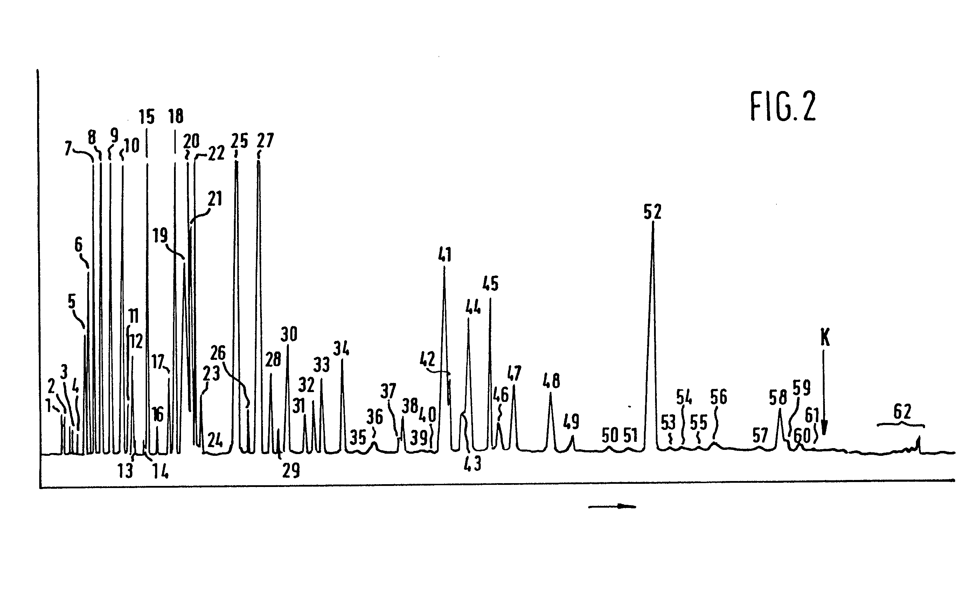

Before benzene would elute from the column 64, the valve 10 is switched from its first position to its second position which is indicated in Figure 1A. Carrier gas is supplied by the line 26 to the port 11 of the valve 10 and exhausted from the valve 10 at the port 13 into the line 34. From the line 34 the carrier gas passes through the port 30 of the valve 32 and is exhausted therefrom at the port 82 into the line 81 and then the column 78. The carrier gas flows through the column 78 into the port 84 of the valve 32 and is exhausted therefrom at the port 86 into the line 90 from which it is provided to the flame ionization detector 88. The column 78 separates the sample by components from $C_1$ through at least $C_8$ as detected by the flame ionization detector 88. The component separation of the sample as detected by the flame ionization detector 88 is indicated in Fig. 2. The peaks identified in Fig. 2 are defined in Table 1.

The horizontal axis of Fig. 2 represents time. The arrow "K" in Fig. 2 represents backflush.

TABLE 1
Identification of Chromatogram (Figure 2)

| Peak Number | Compound |
|---|---|
| 1 | Butanes and Lighter |
| 2 | Isopentane |
| 3 | n-Pentane |
| 4 | 2,2-Dimethylbutane |
| 5 | Cyclopentane |
| 6 | 2,3-Dimethylbutane |
| 7 | 2-Methylpentane |
| 8 | 3-Methylpentane |
| 9 | n-Hexane |
| 10 | Methylcyclopentane |
| 11 | 2,2-Dimethylpentane |
| 12 | 2,4-Dimethylpentane |
| 13 | 2,2,3-Trimethylbutane |
| 14 | Benzene |
| 15 | Cyclohexane |
| 16 | 3,3-Dimethylpentane |
| 17 | 1,1-Dimethylcyclopentane |
| 18 | 2-Methylhexane+2,3-dimethylpentane |
| 19 | 1,cis-3-Dimethylcyclopentane |
| 20 | 3-Methylhexane |
| 21 | 1,trans-3-Dimethylcyclopentane |
| 22 | 1,trans-2-Dimethylcyclopentane |
| 23 | 3-Ethylpentane |
| 24 | 2,2,4-Trimethylpentane |
| 25 | n-Heptane |
| 26 | 1,cis-2-Dimethylcyclopentane |

## TABLE 1 (Cont.)
### Identification of Chromatogram (Figure 2)

| Peak Number | Compound |
|---|---|
| 27 | Methylcyclohexane |
| 28 | 1,1,3-Trimethylcyclopentane |
| 29 | 2,2-Dimethylhexane |
| 30 | Ethylcyclopentane |
| 31 | 2,5-Dimethylhexane |
| 32 | 2,4-Dimethylhexane+2,2,3-trimethylpentane |
| 33 | 1,trans-2,cis-4-Trimethyl-cyclopentane |
| 34 | 1,trans-2,cis-3-Trimethyl cyclopentane |
| 35 | 2,3,4-Trimethylpentane |
| 36 | 1,1,2-Trimethylcyclopentane |
| 37 | 2-Methyl-3-ethylpentane |
| 38 | 2,3-Dimethylhexane |
| 39 | 1,cis-2,trans-4-Trimethyl-cyclopentane |
| 40 | 1,cis-2,trans-3-Trimethyl-cyclopentane |
| 41 | 1,cis-2,cis-4-Trimethylcyclo-pentane+2-methylheptane+3-methyl-3-ethylpentane+3,4-dimethylhexane |
| 42 | 4-methylheptane |
| 43 | 3-Ethylhexane |
| 44 | 3-Methylheptane+1,1-dimethyl-cyclohexane |
| 45 | 1,trans-4-Dimethylcyclohexane+1,cis-3-dimethylcyclohexane |
| 46 | 1-Methyl-cis-3-ethylcyclo-pentane |
| 47 | 1-Methyl-trans-3-ethylcyclo-pentane+1-methyltrans-2-ethylcyclopentane+1-methyl-1-ethylcyclopentane |
| 48 | 1,cis-2,cis-3-Trimethylcyclo-pentane+1,trans-2-dimethyl-cyclohexane |
| 49 | 1,trans-3-Dimethylcyclohexane+1,cis-4-dimethylcyclohexane |
| 50 | $C_8$ Paraffin |
| 51 | Isopropylcyclopentane |
| 52 | n-Octane |
| 53 | $C_8$ Paraffin |
| 54 | 1-Methyl-cis-2-ethylcyclopentane |
| 55 | $C_8$ Paraffin |
| 56 | 1,cis-2-Dimethylcyclohexane |
| 57 | $C_8$ Paraffin |
| 58 | Ethylcyclohexane |
| 59 | n-Propylcyclopentane |
| 60 | $C_8$ Paraffin |
| 61 | $C_8$ Paraffin |
| 62 | $C_9^+$ Saturates |

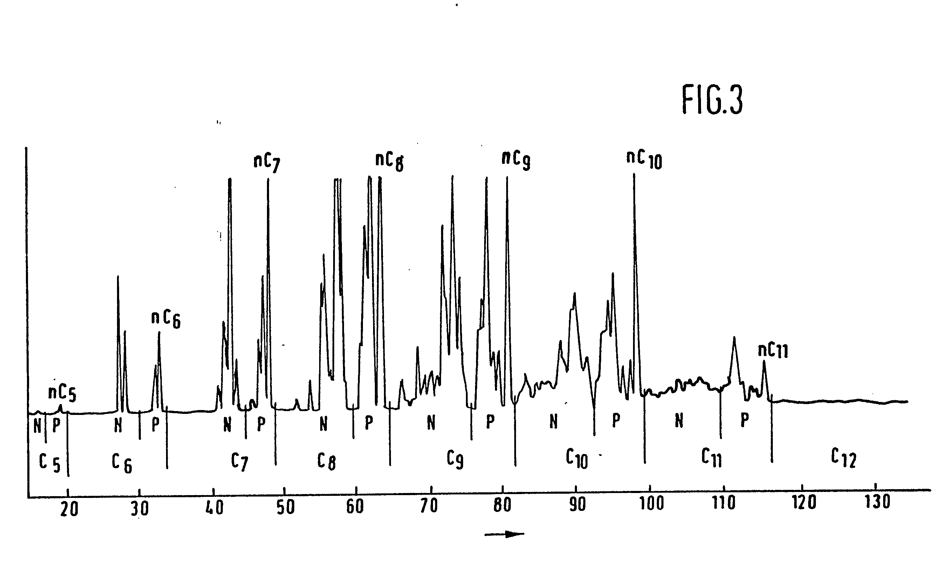

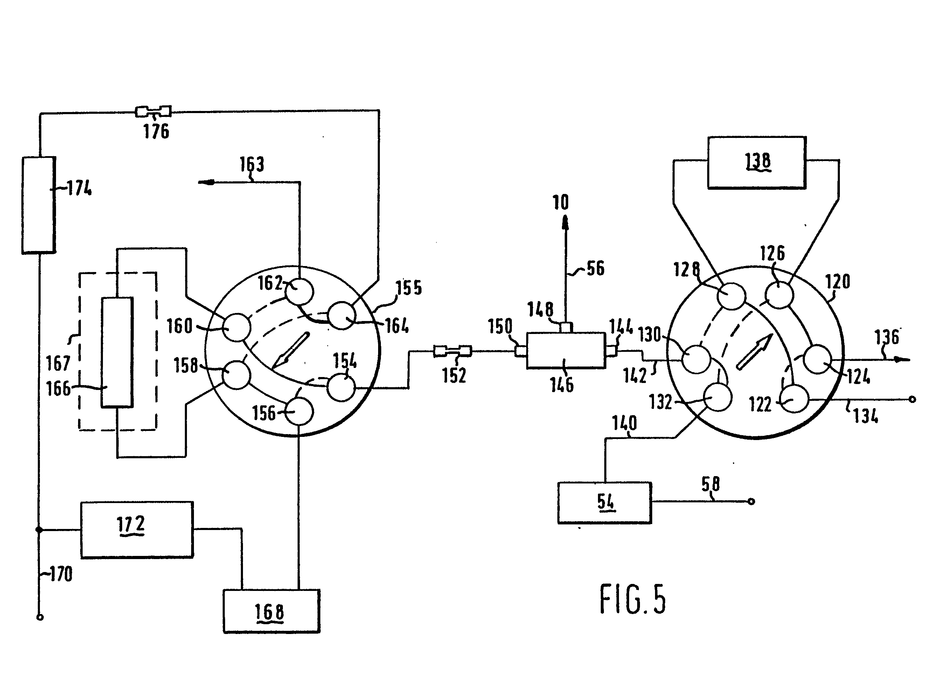

Carrier gas flows from the line 28 into the port 12 of the valve 10 and is exhausted therefrom at the port 14 into the line 38 which provides it to one end of the column 36. The carrier gas passes through the column 36 and is exhausted therefrom to the line 74 from which it is provided to the flame ionization detector 72. At the point at which the valve 10 is switched from the first position to its second position the oven 76 begins its temperature program. The column 36 separates the paraffins and naphthenes in the sample by carbon number as detected by the flame ionization detector 72. Fig. 3 is a typical chromatogram of the separation provided by the column 36. Carrier gas continues to flow through the line 38, the injector 54, the line 56 and the port 18 of the valve 10. The gas is exhausted from the port 16 of the valve 10 into the line 45 from which it enters the port 43 of the splitter 42. In addition, carrier gas from the lines 26 and 28 passes through the line 50 and the valve 44 and the line 52 and the valve 46 respectively to the line 48 from which it enters the port 17 of the valve 10. The carrier gas is exhausted from the valve 10 at the port 15 through the line 37 to the flow restrictor 40 from which it flows through the line 39 into the port 41 of the splitter 42. The combined carrier gases then flow through the port 92 of the splitter 42 into the line 94 to the column 64. It should be noted that this flow is in the opposite direction of the flow through the column 64 when the valve 10 is in its first position; this reversed flow blackflushes the aromatics that were retarded by the column 64 during the time that the sample was injected when the valve 10 was in its first position. The flow backflushes the aromatics from the column 64 into the line 66 to the port 20 of the valve 10 and is exhausted therefrom at the port 19 into the line 62. The line 62 provides the flow to the port 59 of the splitter 60 which then splits a portion of this flow to atmosphere by way of the line 108 and passes the remainder through the port 98 into the line 100. The line 100 provides the flow to the column 96 which separates the aromatics as detected by the flame ionization detector 102 which is connected to the column 96 by the line 104. When the valve 10 is switched from its first position to its second position the oven 106 is progressed through its temperature program. The sample chromatogram obtained with the column 96 is provided in Fig. 4. The horizontal axis represents time (in min). Peak A represents benzene, peak B represents toluene, peaks C represent $C_8$ aromatics and peaks D represent $C_9$ aromatics. At a predetermined time the valve 30 is switched from it first position to its second position so that the flow through the column 78 is reversed to backflush the column 78.

Referring to Fig. 5, an alternative embodiment of the present invention is provided in which an additional branch for detecting an inorganic component of the sample is incorporated with the paraffin, naphthene and aromatic analysis scheme described in reference to Fig. 1. A 6-port valve 120 has ports 122, 124, 126, 128, 130 and 132. When the valve 120 is in its first position the port 122 is connected to the port 128, the port 124 is connected to the port 126 and the port 130 is connected to the port 132. When the valve 120 is in its second position the port 122 is connected to the port 124, the port 126 is connected to the port 132 and the port 128 is connected to the port 130. The port 122 is connected to a line 134 which is adapted for providing a gas sample to the analyzer system. The port 124 is connected to a line 136 which is vented to atmosphere. A variable sample loop 138 is connected across the ports 126 and 128. The port 132 is connected to the injector 54 by a line 140. The injector 54 is the same injector as described hereinabove in reference to Fig. 1, and again it is provided with a carrier gas, such as helium or argon by the line 58. The port 130 is connected by a line 142 to the port 144 of the splitter 146. The port 148 of the splitter 146 is connected to the line 56 which carries a portion of the sample to the port 18 of the valve 10 for analysis as described hereinabove in reference to Fig. 1. The other portion of the sample passes through the port 150 of the splitter 146 through a flow resistor 152 into the port 154 of the valve 155. The valve 155 is a 6-port valve having ports 154, 156, 158, 160, 162 and 164. When the valve 155 is in its first position the port 154 is connected to the port 160, the port 156 is connected to the port 158 and the port 162 is connected to the port 164. When the valve 155 is in its second position the port 154 is connected to the port 156, the port 160 is connected to the port 162 and the port 158 is connected to the port 164. The port 156 is connected to the detector 168 which can be, for example, a thermal conductivity detector for detecting a particular inorganic component of the sample. The column 166 which can be located in an oven 167 is connected across the ports 158 and 160. The port 162 is connected to the line 163 is vented to atmosphere. The port 164 is connected to a valve 174 through a flow restrictor 176. The line 170 which is adapted to be connected to a gas supply means, such as argon, provides gas to both the valve 174 and the flow controller 172. The flow controller 172 provides a reference gas to the detector 168. In this embodiment the valve 120 allows the selection of either a liquid sample injected by the injector 54 or a gas sample provided by the line 134. The flow restrictor 152 is sized to provide the appropriate flow to the column 166. The valve 155 allows the flow through the column 166 to be reversed to backflush the column 166, thereby shortening the analysis time. The flow restrictor 176 modifies the flow to the proper amount for the column 166 during backflushing. The valve 174 is interlocked with the valve 155 so that the valve 174 is closed when the valve 155 is in its first position and the valve 174 is open when the valve 155 is in its second position. If desired the valve 174 and the flow restrictor 176 can be omitted and the valve 155 can be replaced by a four port valve.

Various modifications of the present invention as claimed will become apparent to those skilled in the art from the foregoing description and accompanying drawings.

## Claims

1. An apparatus for analyzing a hydrocarbon sample comprising paraffins, naphthenes and aromatics, comprising means for providing a sample; a first means for supplying gas connected to said sample providing means, a first valve means connected to said sample providing means; a polar column connected to said first valve means for retarding the aromatics of said sample while the remainder passes through; a 13X molecular sieve coated open tubular column (13X MSCOT column) comprising an elongated capillary tube and a layer comprising a plurality of disunited particles of 13X material disposed on the inner surface of the tube by filling the tube with a suspension comprising a dispersive liquid and 13X materials and flowing an inert gas through the tube to dry the tube thereby leaving said layer attached to the inner surface of said tube for separating the paraffins and naphthenes in said sample by carbon number, said 13X MSCOT column having a first end connected to said first valve means and a second end connected to means for determining the amounts of paraffins and naphthenes separated by carbon number by said 13X MSCOT column; means for varying the temperature of said 13X MSCOT column; a mixed binary phase column for separating said sample by components from $C_1$ through at least $C_8$, said mixed binary phase column having a first end connected to said first valve means and a second end connected to means for determining the amounts of said sample separated by components from $C_1$ through at least $C_8$ by said mixed binary phase column; means for maintaining said mixed binary phase column at a predetermined temperature; a nonpolar stationary phase column for separating the aromatics in said sample, said nonpolar stationary phase column having a first end connected to said first valve means and a second end connected to means for determining the amounts of said aromatics separated by said nonpolar stationary phase column; means for varying the temperature of said nonpolar stationary phase column; a second means for supplying gas connected to said first valve means; a third means for supplying gas connected to said first valve means; said first valve means having at least a first and second position, such that in said first position, said polar column is connected to an injecting means, said 13X MSCOT column is connected to said polar column such that a portion of the effluent from said polar column passes through said 13X MSCOT column and said mixed binary phase column is connected to said polar column such that at least a portion of the effluent from said polar column passes through said mixed binary phase column and such that in said second position the flow through said polar column is reversed, said polar column is connected to said first end of said nonpolar stationary column such that the flow from said polar column enters said first end of said nonpolar stationary column, said first end of said 13X MSCOT column is connected to said second gas supply means, and said first end of said mixed binary phase column is connected to said third gas supply means.

2. The apparatus as claimed in claim 1, characterized in that said apparatus further comprises a second valve means connected to said first valve means and said mixed binary phase column, said second valve means having first and second positions such that in said first position said first end of said mixed binary phase column is connected to said first valve means and said second end is connected to said means for determining the amounts of said sample separated by components from $C_1$ through at least $C_8$ by said mixed binary phase column and such that in said second position said second end of said mixed binary phase column is connected to said first valve means and said first end of said mixed binary phase column is connected to said means for determining the amounts of said sample separated by components from $C_1$ through at least $C_8$ by said mixed binary phase column.

3. An apparatus as claimed in claim 1, characterized in that said apparatus further comprises means to switch said first valve means from said first position to said second position at a predetermined time based on the elution time of benzene.

4. The apparatus as claimed in claim 1, characterized in that said apparatus further comprises means connected to said sample providing means for separating a portion of the sample, a first column connected to said separating means for separating a predetermined inorganic component from the sample and means for determining the amount of said predetermined inorganic component separated from said sample by said first column.

5. The apparatus as claimed in claim 4, characterized in that said sample providing means is adapted to provide a liquid sample and wherein said apparatus further comprises means adapted for providing a gas sample and a third valve means connected to said separating means and said liquid sample providing means, said third valve means having at least a first and second position such that in said first position said liquid sample providing means is connected to said separating means and said means for providing a gas sample is not connected to said separating means and such that in said second position said means for providing a gas sample is connected to said separating means and said means for providing a liquid sample is not connected to said separating means.

6. The apparatus as claimed in claim 5, characterized in that said apparatus further comprises a fourth valve means connected to said first column, said fourth valve means having a first position in which flow is directed through said fourth column in a first direction and a second position in which flow is directed through said first column in the opposite direction.

7. The apparatus as claimed in claim 1, characterized in that said apparatus further comprises a fifth valve means having a first end connected to said second means for supplying gas and a second end connected to said first valve means and a sixth valve means having a first end connected to said third gas supply means and a second end connected to said first valve means, said first valve means being adapted such that said fifth and sixth valve means are connected to said nonpolar stationary phase column when said first valve means is in its first position, said fifth and sixth valve means being adapted to be open when said first valve means is in its first position and being adapted to be closed at a predetermined time before said first valve means is switched to said second position.

8. A method of analyzing a hydrocarbon sample comprising paraffins, naphthenes and aromatics, comprising the steps of: injecting a hydrocarbon sample into a first stream of carrier gas; passing the first stream of carrier gas and sample through a polar column having a material for retarding aromatics from the remainder of the sample, retarding in the polar column the aromatics from the sample and allowing the remainder of the sample and the first stream of carrier gas to pass through thereby providing a first effluent from the polar column; splitting the first effluent from the polar column into at least first and second portions; passing the first effluent portion through a 13X molecular sieve coated open tubular column (13X MSCOT column) having a layer comprising a plurality of dis-united particles of 13X material disposed on an inner surface of the column by filling the column with a suspension comprising a dispersive liquid and 13X material and flowing an inert gas through the column thereby leaving a layer comprising a plurality of disunited particles of the 13X material attached to the inner surface of the column for separating paraffins and naphthenes by carbon number; passing the second effluent portion through a mixed binary phase column having a material for separating the second effluent portion by components from $C_1$ through at least $C_8$; separating in said mixed binary phase column the second effluent portion by components from $C_1$ through at least $C_8$; reversing the flow of said first stream of carrier gas through said polar column to remove the aromatics from the polar column thereby providing a second effluent from the polar column; passing the second effluent through a nonpolar stationary phase column having a material for separating the aromatics; separating in said nonpolar stationary phase column the aromatics from the second effluent; providing a second stream of carrier gas to the 13X MSCOT column; providing a third stream of carrier gas to the mixed binary phase column; modifying the temperature of the 13X MSCOT column; modifying the temperature of the nonpolar stationary phase column; detecting the amounts of paraffins and naphthenes separated by carbon number from said sample in said 13X MSCOT column; detecting the amounts of components from $C_1$ through at least $C_8$

separated from said sample in said mixed binary phase column; and detecting the amounts of aromatics separated from said sample in said nonpolar stationary phase column.

9. The method as claimed in claim 8, characterized in that said step of reversing the flow of said first stream of carrier gas through said polar column is performed at a predetermined time related to the elution time of benzene.

10. The method as claimed in claim 8, characterized by the step of reversing the flow through said mixed binary phase column at a predetermined time after the detection of components of said sample from $C_1$ through at least $C_8$.

11. The method as claimed in claim 8, characterized by the steps of: splitting said sample before passing it through said polar column; passing a portion of said split sample through a first column containing a material for separating a predetermined inorganic component from said split sample; separating said predetermined inorganic component in said first column; and detecting the amount of said predetermined inorganic component separated from said portion of the split sample in said first column.

12. The method as claimed in claim 8, characterized by the step of reversing the flow through said first column at a predetermined time.

13. The method as claimed in claim 8, characterized by the step of flowing a gas through said nonpolar stationary phase column until a predetermined period of time before reversing the flow of said first stream of carrier gas through said polar column.

## Patentansprüche

1. Gerät zur Analyse einer Paraffine, Naphthene und Aromaten enthaltenden Kohlenwasserstoffprobe, welches eine Vorrichtung zur Bereitstellung einer Probe; eine mit der Probenbereitstellungsvorrichtung verbundene erste Vorrichtung zur Gasversorgung, ein mit der Probenbereitstellungsvorrichtung verbundene erste Ventileinrichtung; eine mit der ersten Ventileinrichtung verbundene polar Säule zur Zurückhaltung der Aromaten in der Probe, während die verbleibenden Bestandteile durchtreten; eine mit 13X-Molekularsieb beschichtete offene röhrenförmige Säule (13X MSCOT-Säule) mit einer verlängerten Kapillarröhre und einer Schicht, welche eine Vielzahl von getrennten Teilchen aus 13X-Material umfaßt, das auf der inneren Oberfläche der Röhre durch Füllen der Röhre mit einer eine dispersive Flüssigkeit und 13X-Materialien umfassenden Suspension und Durchleiten eines Inertgases durch die Röhre, um die Röhre dadurch zu trocknen, unter Zurücklassung der an der inneren Oberfläche der Röhre angelagerten Schicht verteilt wurde, zur Trennung der Paraffine und Naphthene in der Probe nach der Kohlenstoffzahl, wobei die 13X-MSCOT-Säule ein mit der ersten Ventileinrichtung verbundenes erstes Ende und ein mit einer Vorrichtung zur Bestimmung der Menge an durch die 13X MSCOT-Säule nach der Kohlenstoffzahl getrennten Paraffinen und Naphthenen verbundenes zweites Ende aufweist; eine Vorrichtung zur Regelung der Temperatur der 13X MSCOT-Säule; eine Säule mit gemischter binärer Phase zur Trennung der Probe nach Komponenten von $C_1$ bis wenigstens $C_8$, wobei die Säule mit der gemischten binären Phase ein mit der ersten Ventileinrichtung verbundenes erstes Ende und ein mit einer Vorrichtung zur Bestimmung der durch die Säule mit der gemischten binären Phase nach Komponenten von $C_1$ bis wenigstens $C_8$ getrennten Probe verbundenes zweites Ende aufweist; eine Vorrichtung zum Halten der Säule mit der gemischten binären Phase auf einer vorbestimmten Temperatur; eine Säule mit einer nicht polaren stationären Phase zur Trennung der Aromaten in der Probe, wobei die Säule mit der nicht polaren stationären Phase ein mit der ersten Ventileinrichtung verbundenes erstes Ende und ein mit einer Vorrichtung zur Bestimmung der Menge der in der Säule mit der nicht polaren stationären Phase getrennten Aromaten verbundenes zweites Ende aufweist; eine Vorrichtung zur Variation der Temperatur der Säule mit der nicht polaren stationären Phase; eine zweite mit der ersten Ventileinrichtung zur Gasversorgungsvorrichtung; eine dritte mit dem ersten Ventil verbundene Gasversorgungsvorrichtung; wobei die erste Ventileinrichtung wenigstens eine erste und eine zweite Stellung aufweist, so daß in der ersten Stellung die polare Säule mit einem Injektor verbunden ist, die 13X MSCOT-Säule mit der polaren Säule verbunden ist, so daß ein Teil des Ausflusses aus der polaren Säule durch die 13X MSCOT-Säule tritt und die Säule mit der gemischten binären Phase mit der polaren Säule verbunden ist, so daß wenigstens ein Teil des Ausflusses aus der polaren Säule durch die Säule mit der gemischten binären Phase tritt, und daß in der zweiten Stellung der Fluß durch die polare Säule umgekehrt wird, wobei die polare Säule mit dem ersten Ende der Säule mit der nicht polaren stationären Phase verbunden ist, so daß der Fluß aus der polaren Säule in das erste Ende der Säule mit der nicht polaren stationären Phase eintritt, das erste Ende der 13X MSCOT-Säule mit der zweiten Gasversorgungsvorrichtung verbunden ist und das erste Ende der Säule mit der gemischten binären Phase mit der dritten Gasversorgungsvorrichtung verbunden ist, umfaßt.

2. Gerät, wie in Anspruch 1 beansprucht, dadurch gekennzeichnet, daß das Gerät weiterhin eine mit der ersten Ventileinrichtung verbundene zweite Ventileinrichtung umfaßt, wobei die zweite Ventileinrichtung eine erste und eine zweite Stellung hat, so daß in der ersten Stellung das erste Ende der Säule mit der gemischten binären Phase mit der ersten Ventileinrichtung verbunden ist und das zweite Ende mit der Vorrichtung zur Bestimmung der Menge der durch die Säule mit der gemischten binären Phase nach Komponenten von $C_1$ bis wenigstens $C_8$ aufgetrennten Probe verbunden ist, und das in der zweiten Stellung das zweite Ende der Säule mit der gemischten binären Phase mit der ersten Ventileinrichtung verbunden ist und das erste Ende der Säule mit der gemischten binären Phase mit der Vorrichtung zur Bestimmung der Menge der durch die Säule mit der gemischten binären Phase nach Komponenten von $C_1$ bis wenigstens $V_8$ aufgetrennten Probe verbunden ist.

3. Gerät, wie in Anspruch 1 beansprucht, dadurch gekennzeichnet, daß das Gerät weiterhin eine Vorrichtung zum Umschalten der ersten Ventileinrichtung aus der ersten Stellung in die zweite Stellung zu einer vorbestimmten Zeit auf Basis der Elutionszeit von Benzol umfaßt.

4. Gerät, wie in Anspruch 1 beansprucht, dadurch gekennzeichnet, daß das Gerät weiterhin eine mit der Probenbereitstellungsvorrichtung verbundene Vorrichtung zur Abtrennung eines Teils der Probe, eine mit der Trennvorrichtung verbundene erste Säule zur Abtrennung einer vorbestimmten anorganischen Komponente aus der Probe und eine Vorrichtung zur Bestimmung der Menge der aus der Probe durch die erste Säule abgetrennten vorbestimmten anorganischen Komponente umfaßt.

5. Gerät, wie in Anspruch 4 beansprucht, dadurch gekennzeichnet, daß die Probenbereitstellungsvorrichtung zur Bereitstellung einer flüssigen Probe ausgelegt ist und worin das Gerät weiterhin eine Vorrichtung zur Bereitstellung einer gasförmigen Probe und eine mit der Trennvorrichtung und der Vorrichtung zur Bereitstellung einer flüssigen Probe verbundene dritte Ventileinrichtung umfaßt, wobei die Ventileinrichtung wenigstens über eine erste und eine zweite Stellung verfügt, so daß in der ersten Stellung die Vorrichtung zur Bereitstellung der flüssigen Probe mit der Trennvorrichtung verbunden ist und die Vorrichtung zur Bereitstellung einer gasförmigen Probe nicht mit der Trennvorrichtung verbunden ist, und das in der zweiten Stellung die Vorrichtung zur Bereitstellung einer gasförmigen Probe mit der Trennvorrichtung verbunden ist und die Vorrichtung zur Bereitstellung einer flüssigen Probe nicht mit der Trennvorrichtung verbunden ist.

6. Gerät, wie in Anspruch 5 beansprucht, dadurch gekennzeichnet, daß das Gerät weiterhin eine vierte, mit der ersten Säule verbundene Ventileinrichtung umfaßt, wobei die vierte Ventileinrichtung über eine erste Stellung verfügt, in welcher der Fluß durch die erste Säule in einer ersten Richtung geleitet wird, sowie über eine zweite Stellung, in welcher der Strom durch die erste Säule in entgegengesetzter Richtung geleitet wird.

7. Gerät, wie in Anspruch 1, beansprucht, dadurch gekennzeichnet, daß das Gerät weiterhin eine fünfte Ventileinrichtung mit einem mit der zweiten Gasversorgungsvorrichtung verbundenen ersten Ende und einem mit der ersten Ventileinrichtung verbundenen zweiten Ende sowie eine sechste Ventileinrichtung mit einem mit der dritten Gasversorgungsvorrichtung verbundenen ersten Ende und einem mit der ersten Ventileinrichtung verbundenen zweiten Ende umfaßt, wobei die erste Ventileinrichtung so ausgelegt ist, daß die fünfte und sechste Ventileinrichtung mit der Säule mit der nicht polaren stationären Phase verbunden sind, wenn die erste Ventileinrichtung in der ersten Stellung ist, die fünfte und sechste Ventileinrichtung offengehalten werden, wenn die erste Ventileinrichtung in ihrer ersten Stellung ist und dazu ausgelegt sind, zu einer vorbestimmten Zeit geschlossen wird, bevor die erste Ventileinrichtung in die zweite Stellung geschaltet wird.

8. Verfahren zur Analyse einer Paraffine, Naphthene und Aromaten enthaltenden Kohlenwasserstoffprobe, welches die Schritte umfaßt: Injizieren einer Kohlenwasserstoffprobe in einen ersten Trägergasstrom; Durchleiten des ersten Trägergasstroms mit der Probe durch eine polare Säule mit einem Material zur Zurückhaltung von Aromaten aus den verbleibenden Bestandteilen der Probe; Zurückhalten der Aromaten aus der Probe in der polaren Säule und Passierenlassen der verbleibenden Bestandteile der Probe und des ersten Trägergasstroms und dadurch Bereitstellen eines ersten Ausstroms aus der polaren Säule; Aufspalten des ersten Ausflusses aus der polaren Säule in einen ersten und einen zweiten Teil; Durchleiten des ersten Ausflußteils durch eine offene röhrenförmige Säule mit einer 13X-Molekularsieb-Beschichtung (13X MSCOT-Säule), die eine Schicht mit einer Vielzahl von getrennten Teilchen aus 13X-Material aufweist, welche durch Füllen der Säule mit einer eine dispersive Flüssigkeit und 13X-Material umfassenden Suspension und Durchleiten eines Inertgases durch die Säule auf der inneren Oberfläche der Säule verteilt wurden, wodurch eine auf der inneren Oberfläche der Säule angelagerte Schicht mit einer Vielzahl von getrennten Teilchen aus dem 13X-Material zurückbleibt, zur Auftrennung von Paraffinen und Naphthenen nach der Kohlenstoffzahl; Durchleiten des zweiten Ausflußteils durch eine Säule mit einer gemischen binären Phase mit einem Material zur Auftrennung des zweiten Ausflußteils nach Komponenten von $C_1$ bis wenigstens $C_8$; Trennen des zweiten Ausflußteils nach Komponenten von $C_1$ bis wenigstens $C_8$ in der Säule mit der gemischten binären Phase; Umkehren des Flusses des ersten Trägergasstroms durch die polare Säule zur Entfernung der Aromaten aus der polaren Säule, wodurch ein zweiter Ausfluß aus der polaren Säule ergibt; Durchleiten des zweiten Ausflusses durch eine Säule mit einer nicht polaren stationären Phase mit einem Material zur Abtrennung der Aromaten; Abtrennen der Aromaten aus dem zweiten Ausfluß in der Säule mit der nicht polaren stationären Phase; Bereitstellen eines zweiten Trägergasstroms für die 13X MSCOT-Säule; Bereitstellen eines dritten Trägergasstroms für die Säule mit der gemischten binären Phase; Modifizieren der Temperatur der 13X MSCOT-Säule Modifizieren der Temperatur der Säule mit der nicht polaren stationären Phase; Bestimmen der Menge der in der 13X MSCOT-Säule aus der Probe nach der Kohlenstoffzahl getrennten Paraffine und Naphthene; Bestimmen der Menge der in der Säule mit der gemischten binären Phase aus der Probe getrennten Komponenten von $C_1$ bis wenigstens $C_8$; und Bestimmen der Mengen der aus der Probe in der Säule mit der nicht polaren stationären Phase aus der Probe getrennten Aromaten.

9. Verfahren, wie in Anspruch 8 beansprucht, dadurch gekennzeichnet, daß der Schritt der Umkehr des Flusses des ersten Trägergasstroms durch die polare Säule zu einer vorbestimmten Zeit in Zusammenhang mit der Elutionszeit von Benzol durchgeführt wird.

10. Verfahren, wie in Anspruch 8 beansprucht, gekennzeichnet durch den Schritt der Umkehr des Flusses durch die Säule mit der gemischten binären Phase zu einem vorbestimmten Zeitpunkt nach der Bestimmung der Komponenten der Probe von $C_1$ bis wenigstens $C_8$.

11. Verfahren, wie in Anspruch 8 beansprucht, gekennzeichnet durch die Schritte; Aufspalten der Probe vor dem Durchgang durch die polare Säule; Durchleiten eines Teils der aufgespalteten Probe durch eine erste Säule, die ein Material zur Abtrennung einer vorbestimmten anorganischen Komponente aus der aufgespalteten Probe enthält; Abtrennen der vorbestimmten anorganischen Komponenten in der ersten Säule; und Bestimmen der Menge der aus diesem Teil der aufgespalteten Probe in der ersten Säule abgetrennten vorbestimmten anorganischen Komponente.

12. Verfahren wie in Anspruch 8 beansprucht, gekennzeichnet durch den Schritt der Umkehr des Flusses durch die erste Säule zu einer vorbestimmten Zeit.

13. Verfahren, wie in Anspruch 8 beansprucht, gekennzeichnet durch den Schritt des Durchleitens eines Gases durch die Säule mit der nicht polaren stationären Phase bis zu einer vorbestimmten Zeit vor der Umkehr des Flusses des ersten Trägergasstroms durch die polare Säule.

## Revendications

1. Appareil pour l'analyse d'un échantillon d'hydrocarbures comprenant des paraffines, des naphtènes et des aromatiques, qui comprend un moyen d'amenée d'un échantillon; un premier moyen d'arrivée de gaz relié audit moyen d'amenée de l'échantillon, un premier moyen de soupape relié audit moyen d'amenée de l'échantillon; une colonne polaire reliée audit premier moyen de soupape pour retarder les aromatiques dudit échantillon tandis que le reste passe; une colonne tubulaire ouverte, recouverte d'un tamis moléculaire 13X (colonne 13X MSCOT) comprenant un tube capillaire allongé et une couche comprenant plusieurs particules désunies du matériau 13X disposées sur la surface intérieure du tube par remplissage du tube avec une suspension comprenant un liquide dispersif et des matériaux 13X et écoulement d'un gaz inerte à travers le tube pour sécher le tube ce qui laisse ainsi ladite couche fixée sur la surface intérieure dudit tube pour séparer les paraffines et les naphtènes dans ledit échantillon par le nombre de carbones, ladite colonne 13X MSCOT ayant une première extrémité reliée audit premier moyen de soupape et une seconde extrémité reliée au moyen pour déterminer les quantités de paraffines et de naphtènes séparées par le nombre de carbones par ladite colonne 13X MSCOT; un moyen pour faire varier la température de ladite colonne 13X MSCOT; une colonne à phase binaire mixte pour séparer ledit échantillon en constituants allant de $C_1$ jusqu'à au moins $C_8$, ladite colonne à phase binaire mixte ayant une première extrémité reliée audit premier moyen de soupape et une seconde extrémité reliée au moyen pour déterminer les quantités dudit échantillon séparées en constituants allant de $C_1$ jusqu'à au moins $C_8$ par ladite colonne à phase binaire mixte; un moyen pour maintenir ladite colonne à phase binaire mixte à une température prédéterminée; une colonne à phase stationnaire non polaire pour séparer les aromatiques dans ledit échantillon, ladite colonne à phase stationnaire non polaire ayant une première extrémité reliée audit premier moyen de soupape et une seconde extrémité reliée au moyen pour déterminer les quantités desdits aromatiques séparées par ladite colonne à phase stationnaire non polaire; un moyen pour faire varier la température de ladite colonne à phase stationnaire non polaire; un second moyen d'arrivée de gaz relié audit premier moyen de soupape; un troisième moyen d'arrivée de gaz relié audit premier moyen de soupape; ledit premier moyen de soupape ayant au moins une première et une seconde positions, de telle sorte que dans ladite première position, ladite colonne polaire est reliée à un moyen d'injection, ladite colonne 13X MSCOT est reliée à ladite colonne polaire de telle manière qu'une portion de l'effluent provenant de ladite colonne polaire traverse ladite colonne 13X MSCOT et ladite colonne à phase binaire mixte est reliée à ladite colonne polaire de telle manière qu'au moins une portion de l'effluent provenant de ladite colonne polaire traverse ladite colonne à phase binaire mixte, et de telle sorte que dans ladite seconde position, l'écoulement à travers ladite colonne polaire est inversé, ladite colonne polaire est reliée à ladite première extrémité de ladite colonne stationnaire non polaire de telle manière que le courant provenant de ladite colonne polaire pénètre dans ladite première extrémité de ladite colonne stationnaire non polaire, ladite première extrémité de ladite colonne 13X MSCOT est reliée audit second moyen d'arrivée de gaz, et ladite première extrémité de ladite colonne à phase binaire mixte est reliée audit troisième moyen d'arrivée de gaz.

2. Appareil selon la revendication 1, caractérisé en ce que ledit appareil comprend en outre un second moyen de soupape relié audit premier moyen de soupape et à ladite colonne à phase binaire mixte, ledit second moyen de soupape ayant une première et une seconde positions de telle sorte que dans ladite première position, ladite première extrémité de ladite colonne à phase binaire mixte est reliée audit premier moyen de soupape et ladite seconde extrémité est reliée audit moyen pour déterminer les quantités dudit échantillon séparées en constituants allant de $C_1$ jusqu'à au moins $C_8$ par ladite colonne à phase binaire mixte, et de telle sorte que dans ladite seconde position, ladite seconde extrémité de ladite colonne à phase binaire mixte est reliée audit premier moyen de soupape et ladite première extrémité de ladite colonne à phase binaire mixte est reliée audit moyen pour déterminer les quantités dudit échantillon séparées en constituants allant de $C_1$ jusqu'à au moins $C_8$ par ladite colonne à phase binaire mixte.

3. Appareil selon la revendication 1, caractérisé en ce que ledit appareil comprend en outre un moyen pour commuter ledit premier moyen de soupape de ladite première position à ladite seconde position, à un temps prédéterminé basé sur le temps d'élution du benzène.

4. Appareil selon la revendication 1, caractérisé en ce que ledit appareil comprend en outre un moyen relié audit moyen d'amenée de l'échantillon pour séparer une portion de l'échantillon, une première colonne reliée audit moyen de séparation pour séparer un constituant inorganique prédéterminé de l'échantillon et un moyen pour déterminer la quantité dudit constituant inorganique prédéterminé séparée dudit échantillon par ladite première colonne.

5. Appareil selon la revendication 4, caractérisé en ce que ledit moyen d'amenée de l'échantillon est adapté pour fournir un échantillon liquide, et en ce que ledit appareil comprend en outre un moyen adapté pour fournir un échantillon gazeux et un troisième moyen de soupape relié audit moyen de séparation et audit moyen d'amenée de l'échantillon liquide, ledit troisième moyen de soupape ayant au moins une première et un seconde positions de telle sorte que dans ladite première position, ledit moyen d'amenée de l'échantillon liquide est reliée audit moyen de séparation et ledit moyen d'amenée d'un échantillon gazeux n'est pas relié audit moyen de séparation, et de telle sorte que dans ladite seconde position, ledit moyen d'amenée d'un échantillon gazeux est relié audit moyen de séparation et ledit moyen d'amenée d'un échantillon liquide n'est pas relié audit moyen de séparation.

6. Appareil selon la revendication 5, caractérisé en ce que ledit appareil comprend en outre un quatrième moyen de soupape relié à ladite première colonne, ledit quatrième moyen de soupape ayant une première position dans laquelle le courant est dirigé à travers ladite quatrième colonne dans une première direction et une seconde position dans laquelle le courant est dirigé à travers ladite colonne dans la direction opposée.

7. Appareil selon la revendication 1, caractérisé en ce que ledit appareil comprend en outre un cinquième moyen de soupape ayant une première extrémité reliée audit second moyen d'arrivée de gaz et une seconde extrémité reliée audit premier moyen de soupape, et un sixième moyen de soupape ayant une première extrémité reliée audit troisième moyen d'arrivée de gaz et une seconde extrémité reliée audit premier moyen de soupape, ledit premier moyen de soupape étant adapté pour que lesdits cinquième et sixième moyens de soupape soient reliés à ladite colonne à phase stationnaire non polaire lorsque ledit premier moyen de soupape est dans sa première position, lesdits cinquième et sixième moyens de soupape étant adaptés pour être ouverts lorsque ledit premier moyen de soupape est dans sa première position et étant adaptés pour être fermés, à un temps prédéterminé, avant la commutation dudit premier moyen de soupape dans sa seconde position.

8. Procédé pour l'analyse d'un échantillon d'hydrocarbures comprenant des paraffines, des naphthènes et des aromatiques, qui comprend les étapes de: injection d'un échantillon d'hydrocarbures dans un premier courant de gaz vecteur; passage du premier courant de gaz vecteur et de l'échantillon à travers une colonne polaire ayant un matériau pour retarder les aromatiques par rapport au reste de l'échantillon; ralentissement dans la colonne polaire des aromatiques de l'échantillon et passage libre à travers celle-ci du reste de l'échantillon et du premier courant de gaz vecteur, ce qui fournit ainsi un premier effluent provenant de la colonne polaire; partage du premier effluent provenant de la colonne polaire en au moins une première et une seconde portions; passage de la première portion de l'effluent à travers une colonne tubulaire ouverte recouverte d'un tamis moléculaire 13X (colonne 13X MSCOT) ayant une couche comprenant plusieurs particules désunies du matériau 13X disposées sur une surface intérieure de la colonne par remplissage de la colonne avec une suspension comprenant un liquide dispersif et la matériau 13X et écoulement d'un gaz inerte à travers la colonne ce qui laisse ainsi une couche comprenant plusieurs particules désunies du matériau 13X fixées à la surface intérieure de la colonne pour séparer les paraffines et les naphtènes par leur nombre de carbones; passage de la seconde portion de l'effluent à travers une colonne à phase binaire mixte ayant un matériau pour séparer la seconde portion de l'effluent en constituants allant de $C_1$ jusqu'à au moins $C_8$; séparation dans ladite colonne à phase binaire mixte de la seconde portion de l'effluent en constituants allant de $C_1$ jusqu'à au moins $C_8$; inversion de l'écoulement dudit premier courant de gaz vecteur à travers ladite colonne polaire pour enlever les aromatiques de la colonne polaire en fournissant ainsi un second effluent provenant de la colonne polaire; passage du second effluent à travers une colonne à phase stationnaire non polaire ayant un matériau pour séparer les aromatiques; séparation dans ladite colonne à phase stationnaire non polaire des aromatiques provenant du second effluent; amenée d'un second courant de gaz vecteur vers la colonne 13X MSCOT; amenée d'un troisième courant de gaz vecteur vers la colonne à phase binaire mixte;

modification de la température de la colonne 13X MSCOT; modification de la température de la colonne à phase stationnaire non polaire; détection des quantités de paraffines et de naphtènes séparées par le nombre de carbones dans ledit échantillon dans ladite colonne 13X MSCOT; détection des quantités des constituants allant de $C_1$ jusqu'à au moins $C_8$, séparées dans ledit échantillon dans ladite colonne à phase binaire mixte; et détection des quantités d'aromatiques séparées dans ledit échantillon dans ladite colonne à phase stationnaire non polaire.

9. Procédé selon la revendication 8, caractérisé en ce que l'on effectue ladite étape d'inversion de l'écoulement dudit premier courant de gaz vecteur à travers ladite colonne polaire à un temps prédéterminé par rapport au temps d'élution du benzène.

10. Procédé selon la revendication 8, caractérisé en ce que l'on effectue l'étape d'inversion de l'écoulement à travers ladite colonne à phase binaire mixte, à un temps prédéterminé après la détection des constituants dudit échantillon, en $C_1$ jusqu'à au moins $C_8$.

11. Procédé selon la revendication 8, caractérisé par les étapes de: partage dudit échantillon avant son passage à travers ladite colonne polaire; passage d'une portion dudit échantillon fractionné à travers une première colonne contenant un matériau pour séparer un constituant inorganique prédéterminé dudit échantillon fractionné; séparation dudit constituant inorganique prédéterminé dans ladite première colonne; et détection de la quantité dudit constituant inorganique prédéterminé séparé de ladite portion de l'échantillon fractionné dans ladite première colonne.

12. Procédé selon la revendication 8, caractérisé par l'étape d'inversion de l'écoulement à travers ladite première colonne à un temps prédéterminé.

13. Procédé selon la revendication 8, caractérisé par l'étape d'écoulement d'un gaz à travers ladite colonne à phase stationnaire non polaire jusqu'à une période prédéterminée de temps avant l'inversion de l'écoulement dudit premier courant de gaz vecteur à travers ladite colonne polaire.

FIG.1

EP 0 127 908 B1

FIG.1A

FIG.4

# FIG. 2

FIG.3

EP 0 127 908 B1

FIG.5

EP 0 127 908 B1